# EUROPEAN PATENT SPECIFICATION

(11) **EP 3 568 694 B1**
(45) Date of publication and mention of the grant of the patent: **16.02.2022**
(21) Application number: 18736294.2
(22) Date of filing: 09.01.2018
(51) Int. Cl.: A61K 47/69, A61K 47/64, G01N 33/48, A61K 33/24, A61K 38/08, A61K 47/54, A61P 35/00, A61K 47/62, A61K 31/704, A61K 33/242

(54) **TARGETED DOXORUBICIN-GOLD NANOCONJUGATES FOR TUMOR THERAPY**
GEZIELTE DOXORUBICIN-GOLD NANOKONJUGATE FÜR TUMORTHERAPIE
NANOCONJUGUÉS DE DOXORUBICINE-OR CIBLÉS POUR LA THÉRAPIE ANTITUMORALE

(30) Priority: 09.01.2017 US 201762444114 P
(43) Date of publication of application: 20.11.2019
(73) Proprietor: The Curators of the University of Missouri, Columbia, MO 65211 (US)
(72) Inventor: KANNAN, Raghuraman, Columbia Missouri 65211 (US); ZAMBRE, Ajit, Columbia Missouri 65211 (US); UPENDRAN, Anandhi, Columbia Missouri 65211 (US)
(74) Representative: Taruttis, Stefan Georg
(86) International application number: PCT/US2018/012882
(87) International publication number: WO 2018/129501

(56) References cited:
- WO-A1-2015/103028
- US-A1- 2013 022 682
- US-A1- 2013 138 032

## Description

### FIELD OF DISCLOSURE

The present disclosure relates to doxorubicin-gold nanoconjugates that can be used for cancer treatment. The disclosure also relates to methods of making and using the nanoconjugates.

### BACKGROUND

Doxorubicin (DOX) is a chemotherapeutic drug currently in clinical use for several tumors including breast cancer, ovarian cancer, liver cancer, kidney cancer, and others.

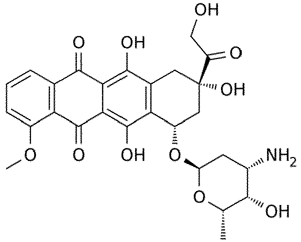

### Doxorubicin

One of the major side effects associated with doxorubicin is its cardiotoxicity. Cardiotoxicity can be minimized by targeted administration or by lowering concentration of the drug. However, reducing the quantity of drug administered correspondingly reduces cytotoxicity and efficacy. Antibody-drug conjugates have been developed for targeted delivery, but there is still a need to develop a doxorubicin delivery platform with low cardiotoxicity and/or high efficacy. US 2013/138032 A1 describes a pH-sensitive gold-nanoparticle -doxorubicin conjugate

### SUMMARY

The present disclosure provides gold nanoconjugates for targeted delivery of doxorubicin. The present disclosure provides a nanoconjugate comprising: a gold nanoparticle (AuNP), dithiolated diethylenetriamine pentaacetic acid (DTDTPA), a thioctic acid terminated peptide, and doxorubicin, wherein the DTDTPA is directly linked to the gold nanoparticle surface via at least one Au-S bond, the thioctic acid terminated peptide is directly linked to the gold nanoparticle surface via at least one Au-S bond, and doxorubicin is linked to the DTDTPA.

In some embodiments, the thioctic acid terminated peptide is thioctic bombesin. In some embodiments, the thioctic bombesin has a sequence: Lipoic-Gln-Trp-Ala-Val-Gly-His-Leu- Met-NH₂.

In some embodiments, the doxorubicin is linked to the DTDTPA via an amide bond.

The present disclosure also provides a process for preparing a nanoconjugate disclosed herein, wherein the process comprises conjugating an AuNP-DTDTPA nanoparticle with the thioctic acid terminated peptide, wherein the gold in the gold nanoparticle and the thioctic acid terminated peptide are present in a stoichiometric ratio of about 1:0.5 to about 1:4. In some embodiments, the gold in the gold nanoparticle and the thioctic acid terminated peptide are present in a stoichiometric ratio of about 1:2.

In some embodiments, the nanoconjugate disclosed herein comprises about 1% to about 40% of the thioctic acid terminated peptide by weight of the nanoconjugate. In some embodiments, the nanoconjugate disclosed herein comprises about 20% to about 30% of the thioctic acid terminated peptide by weight of the nanoconjugate.

In some embodiments, the nanoconjugate disclosed herein comprises about 0.01% to about 0.05% of doxorubicin by weight of the nanoconjugate. In some embodiments, the nanoconjugate disclosed herein comprises about 0.03% of doxorubicin by weight of the nanoconjugate.

In some embodiments, the nanoconjugate has a hydrodynamic diameter of about 110 nm to about 140 nm as measured by dynamic light scattering (DLS). In some embodiments, the nanoconjugate has a hydrodynamic diameter of about 120 nm to about 130 nm as measured by dynamic light scattering (DLS).

In some embodiments, the nanoconjugate has a zeta potential value of about -15 mV to about -30 mV. In some embodiments, the nanoconjugate has a zeta potential value of about -20 mV to about -25 mV.

The present disclosure also provides a pharmaceutical composition comprising the nanoconjugate disclosed herein and a pharmaceutically acceptable carrier.

The present disclosure further provides compounds for use in a method for treating a cancer comprising administering a therapeutically effective amount of the nanoconjugate disclosed herein to a subject in need thereof.

In some embodiments, the cancer is selected from the group consisting of acute lymphoblastic leukemia (ALL), acute myeloblastic leukemia (AML), bone sarcoma, breast cancer, endometrial cancer, gastric cancer, head and neck cancer, Hodgkin lymphoma, Non-Hodgkin lymphoma, liver cancer, kidney cancer, multiple myeloma, neuroblastoma, ovarian cancer, lung cancer, soft tissue sarcoma, thyomas, thyroid cancer, bladder cancer, uterine sarcoma, prostate cancer, colon cancer, ovarian cancer, non-small cell lung cancer, pancreatic cancer, Wilms' tumor, and Waldenstrom macroglobulinemia.

In some embodiments, the nanoconjugate is administered to the subject by injection.

### BRIEF DESCRIPTION OF THE DRAWINGS

The following is a brief description of the drawings which are presented for the purposes of illustrating the exemplary embodiments disclosed herein and not for the purposes of limiting the same.
FIG. 1 shows synthesis of BBN-AuNP-DTDTPA-DOX (NP2-DOX).
FIG. 2 shows a schematic structure of BBN-AuNP-DTDTPA-DOX (NP2-DOX).
FIG. 3 shows a graph of thioctic acid terminated bombesin (TA-BBN) titration with [AuNP(DTDTPA)] to determine the amount of TA-BBN to be accommodated by [AuNP(DTDTPA)] as measured by HPLC. Stichiometric ratios of Au:BBN are presented in the bracket.
FIGs. 4A and 4B show TEM images of nanoconjugates NP1 and NP2, respectively. FIG. 3C shows UV-visible absorption spectrum of nanoconjugates NP1 and NP2.
FIG. 5A shows XPS survey spectrum of nanoconjugate NP2. FIG. 5B shows XPS hi-resolution spectrum of the C1s region of nanoconjugate NP2.
FIG. 6A shows XPS hi-resolution spectrum of the S2p region of nanocontruct NP2. FIG. 6B shows XPS hi-resolution spectrum of the Au4f region of nanocontruct NP2.
FIG. 7 shows fluorescence spectrum of doxorubicin when excited at 580 nm and emission at 590 nm.
FIG. 8 shows estimation of doxorubicin loading on nanoconjugates NP1 and NP2 by fluorescence spectroscopy.
FIG. 9 shows fluorescence spectrum of nanoconjugates NP1 and NP1-DOX.
FIG. 10 shows fluorescence spectrum of nanoconjugates NP2 and NP2-DOX.
FIG. 11 shows UV-Visible absorption spectrum of NP1, NP2, NP1-DOX, and NP2-DOX.
FIGs. 12A and 12 B show *in vitro* stability studies of nanoconjugates NP1 and NP2 by UV-visible absorption spectroscopy. FIGs. 12C and 12D show *in vitro* stability studies of nanoconjugates NP1 and NP2 by hydrodynamic size. FIGs. 12E and 12F show *in vitro* stability studies of nanoconjugates NP1 and NP2 by zeta potential when incubated in various biologically relevant solutions.
FIG. 13 shows IC₅₀ values of nanoconjugate NP2 at various stoichiometric ratios of Au:BBN (1:0.4, 1:2, 1:4) against ¹²⁵I-BBN in GRP receptors expressing prostate tumor PC-3 cells.
FIG. 14 shows *in vitro* cytotoxicity of doxorubicin alone, NP1-DOX, and NP2-DOX in non-GRP expressing MDA-MB-231 human breast cancer cells.
FIG. 15 compares the effect of NP1-DOX, NP2-DOX with Doxorubicin alone in non-GRP expressing human breast cancer cells.
FIG. 16 shows *in vitro* cytotoxicity of doxorubicin alone, NP1-DOX, and NP2-DOX in GRP expressing PC3 human prostate cancer cells.
FIG. 17 compares the effect of NP1-DOX, NP2-DOX with Doxorubicin alone in GRP expressing human prostate cancer cells.
FIG. 18 compares IC₅₀ values of doxorubicin in the nanoconjugates (NP1-DOX and NP2-DOX) with doxorubicin alone in GRP expressing (PC3) human cancer cells.
FIG. 19 compares IC₅₀ values of doxorubicin in the nanoconjugates (NP1-DOX and NP2-DOX) with Doxorubicin alone in non-GRP expressing (MDA-MB-231) human cancer cells.
FIG. 20 compares the difference of cell death percentage for NP1-DOX and NP2-DOX in GRP expressing (PC3) and non-GRP expressing (MDA-MB-231) human cancer cells

### DETAILED DESCRIPTION

Gastrin releasing peptide (GRP) receptors are overexpressed in a variety of human cancer cells, such as breast cancer, prostate cancer, colon cancer, and other cancers. Bombesin peptide and its analogs are known to target GRP receptors. In certain embodiments, the present disclosure provides a multicomponent nanoparticle delivery system (nanoconjugate) comprising gold nanoparticles (AuNP), a targeting agent (e.g. bombesin (BBN)), and doxorubicin (DOX) as a cancer therapeutic agent. The gold nanoparticles are stabilized with dithiolated diethylenetriamine pentaacetic acid (DTDTPA) and can have a multi-layered structure. Thus, in certain embodiments, the nanoconjugate can be BBN-AuNP(DTDTPA)-DOX. *In vitro* cytotoxicity assays performed in breast cancer cells surprisingly demonstrated that BBN-AuNP(DTDTPA)-DOX is much more potent as compared to free doxorubicin.

Various examples and embodiments of the inventive subject matter disclosed here are possible and will be apparent to a person of ordinary skill in the art, given the benefit of this disclosure. In this disclosure reference to "some embodiments," "certain embodiments," and similar phrases each means that those embodiments are non-limiting examples of the inventive subject matter, and there are alternative embodiments which are not excluded.

The articles "a," "an," and "the" are used herein to refer to one or more than one (*i.e.,* to at least one) of the grammatical objects of the article. By way of example, "an element" means one element or more than one element. The term "or" means "and/or". The terms "comprising", "having", "including", and "containing" are to be construed as open-ended terms (i.e., meaning "including, but not limited to").

The word "comprising" is used in a manner consistent with its open-ended meaning, that is, to mean that a given product or process can optionally also have additional features or elements beyond those expressly described. It is understood that wherever embodiments are described with the language "comprising," otherwise analogous embodiments described in terms of "consisting of" and/or "consisting essentially of" are also contemplated and within the scope of this disclosure.

As used herein, the term "about" means ±10% of the noted value. By way of example only, a composition comprising "about 30 weight percent" of a compound could include from 27 weight percent of the compound up to and including 33 weight percent of the compound.

As used herein, the term "nanoconjugate" refers to a nanomaterial comprising a gold nanoparticle and other components, such as a stabilizing agent, a targeting agent, and a therapeutic agent. In certain embodiments, the stabilizing agent and targeting agent can be directly linked to the gold nanoparticle surface via one or more covalent or non-covalent bonds. Some components, such as the therapeutic agent, can be linked to the gold nanoparticle through another component, such as the stabilizing agent.

As used herein, the term "therapeutically effective amount" means an amount effective, when administered to a patient, to provide the intended therapeutic benefit. The amount that is "effective" may vary from subject to subject, depending on the age and general condition of the individual, and the like.

In certain embodiments, the present disclosure provides a nanoconjugate comprising: a gold nanoparticle (AuNP), dithiolated diethylenetriamine pentaacetic acid (DTDTPA), a thioctic acid terminated peptide, and doxorubicin, wherein the DTDTPA is directly linked to the gold nanoparticle surface via at least one Au-S bond, the thioctic acid terminated peptide is directly linked to the gold nanoparticle surface via at least one Au-S bond, and doxorubicin is linked to the DTDTPA.

In certain embodiments, the nanoconjugate disclosed herein consists essentially of: a gold nanoparticle (AuNP), dithiolated diethylenetriamine pentaacetic acid (DTDTPA), a thioctic acid terminated peptide, and doxorubicin, wherein the DTDTPA is directly linked to the gold nanoparticle surface via at least one Au-S bond, the thioctic acid terminated peptide is directly linked to the gold nanoparticle surface via at least one Au-S bond, and doxorubicin is linked to the DTDTPA.

Gold nanoparticles stabilized with DTDTPA, *i.e.*, AuNP-DTDTPA, are known in the art. For example, WO 2015/103028 and Debouttiere et al., Advan. Funt. Mater. 2006, 16:2330-39 describe preparation and use of AuNP-DTDTPA.

The chemical structure of DTDTPA is shown below. DTDTPA molecules can be linked to the gold nanoparticule surface via one or two Au-S bonds.

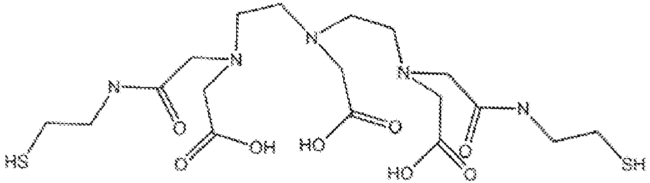

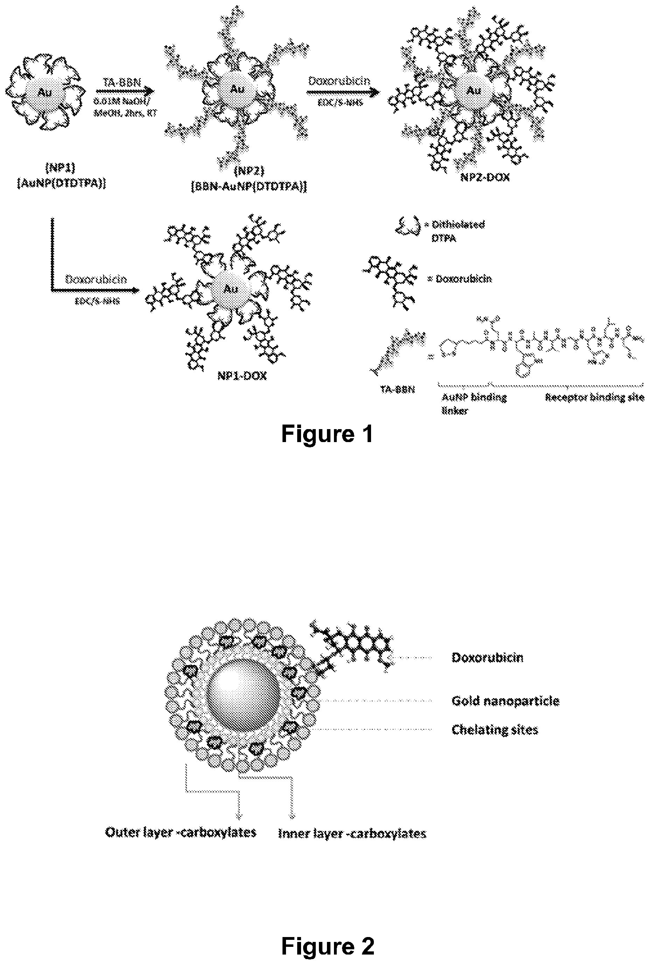

Without wishing to be bound theory, it is believed that DTDTPA loops surrounding the AuNP surface can include one, two, three, four, five, or more DTDTPA molecules. For example, in some embodiments and again without wishing to be bound to a particular theory, it is believed that both thiol groups of a given DTDTPA molecule can both form an Au-S bond to the AuNP surface, thus forming a loop with one DTDTPA molecule. In another embodiment, one thiol group of a given DTDTPA molecule can form an Au-S bond to the AuNP surface, while the second thiol group can form an intermolecuar disulfide bond (S-S) with a second DTDTPA molecule. The second DTDTPA molecule can either be linked to still another DTDTPA molecule via a disulfide bond, or can be linked to the the AuNP surface via an Au-S bond thus forming of a loop consisting of two or more DTDTPA molecules. Depending upon how the DTDTPA molecules arrange themselves, the stabilized nanoparticles can contain a multilayered organic shell of penta-acetic acid molecules on the surface of AuNPs. *See* Debouttiere et al., Advan. Funt. Mater. 2006, 16:2330-39; and **Figure 2**.

In some embodiments, the nanoconjugate disclosed herein comprises about 10% to about 60% of DTDTPA by weight of the nanoconjugate. In some embodiments, the nanoconjugate comprises about 10%, about 15%, about 20%, about 25%, about 30%, about 35%, about 40%, about 41%, about 42%, about 43%, about 44%, about 45%, about 46%, about 47%, about 48%, about 49%, about 50%, about 51%, about 52%, about 53%, about 54%, about 55%, or about 60% of DTDTPA by weight of the nanoconjugate, e.g., about 45%, or a range between any two of the preceeding values, e.g., about 30% to about 60%, or about 40% to about 50%. In some embodiments, the nanoconjugate comprises about 45% of DTDTPA by weight of the nanoconjugate.

In typical embodiments, and as discussed above, the nanoconjugate disclosed herein can comprise a thioctic acid terminated peptide as a targeting agent. In certain embodiments, the targeting peptide can be bombesin peptide. Bombesin peptide can be used to functionalize gold nanomaterials for targeting certain tumor cells. *See* Chanda et al., Nano Lett. 2009, 9(5): 1798-1805; Chanda et al. PNAS 2010, 107(19): 8760-8765; Suresh et al., Bioconjuate Chem. 2014, 25, 1565-1579; and Silva et al., Bioconjuate Chem. 2016, 27, 1153-1164. The 14-amino acid peptide bombesin isolated from the skin amphibian Bombina and related gastrin-releasing peptides (GRP) exhibit an enhanced response in a variety of tumor tissues, e.g., in small cell lung, prostate, breast, and colon cancer. It has been reported that analogs of bombesin with modified structures exhibited a similar or even higher affinity for the GRP receptors. *See e.g.,* Chanda et al., Nano Lett. 2009, 9(5): 1798-1805 and references cited therein. In certain embodiments described herein, a seven-amino acid truncated bombesin analogue (BBN), shown in Figure 1, was used as a targeting agent.

In some embodiments, the thioctic acid terminated peptide can be thioctic terminated bombesin (TA-BBN, alternatively referred to as BBN herein) having a sequence: Lipoic-Gln-Trp-Ala-Val-Gly-His-Leu-Met-NH₂.

In some embodiments, the thioctic acid terminated peptide (e.g., TA-BBN) can be directly linked to the gold nanoparticle surface via one or two Au-S bonds.

In some embodiments, the nanoconjugate disclosed herein comprises about 1% to about 40% of thioctic acid terminated peptide by weight of the nanoconjugate. In some embodiments, the nanoconjugate comprises about 1%, about 5%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 35%, or about 40% of thioctic acid terminated peptide by weight of the nanoconjugate, e.g., about 20% or about 25%, or a range between any two of the preceeding values, e.g., about 10% to about 30%, about 20% to about 30%, or about 15% to about 25%. In some embodiments, the nanoconjugate comprises about 20% or about 25% of thioctic acid terminated peptide by weight of the nanoconjugate.

In some embodiments, the AuNP-DTDTPA-BBN construct, which can be subsequently linked with doxorubicin, can be prepared by a process comprising conjugating AuNP-DTDTPA nanoparticle with the thioctic acid terminated peptide, wherein the gold in the gold nanoparticle and the thioctic acid terminated peptide can be present in in the conjugation reaction in a stoichiometric ratio of about 1:0.5 to about 1:4. The stoichiometric ratio used herein referes to a molar ratio.

In some embodiments, the stoichiometric ratio of the gold in the gold nanoparticle to the thioctic acid terminated peptide used in the conjugation reaction can be about 1:0.75, about 1:1, about 1:1.5, about 1:2, about 1:2.5, about 1:3, about 1:3.5, or about 1:4, *e.g.,* about 1:2, or a range between any two of the preceeding ratios, *e.g.*, about 1:1 to about 1:4, about 1:1 to about 1:3, about 1:1 to about 1:2, about 1:1.5 to about 1:4, about 1:1.5 to about 1:3, about 1:1.5 to about 1:2.5, or about 1:1.5 to about 1:2.

In some embodiments, the amount of the thioctic acid terminated peptide used in the conjugation reaction is in a ratio with the gold in the gold nanoparticle of at least about 1:1.5, at least about 1:2, at least about 1:2.5, or at least about 1:3.

In some embodiments, the stoichiometric ratio of the gold in the gold nanoparticle to the thioctic acid terminated peptide used in the conjugation reaction is about 1:2.

Typically, the doxorubicin in the nanoconjugate is linked to the DTDTPA via an amide bond, formed between a carboxylate group of the DTDTPA and the amino group of the doxorubicin.

In some embodiments, the nanoconjugate disclosed herein comprises about 0.01% to about 0.05% of doxorubicin by weight of the nanoconjugate. In some embodiments, the nanoconjugate can comprise about 0.01%, about 0.015%, about 0.02%, about 0.025%, about 0.03%, about 0.035%, about 0.04%, about 0.045%, or about 0.05% of doxorubicin by weight of the nanoconjugate, *e.g.,* about 0.03%, or a range between any two of the preceeding values, *e.g.,* about 0.01% to about 0.04%, about 0.01% to about 0.03%, about 0.02% to about 0.05%, about 0.02% to about 0.04%, about 0.02% to about 0.03%, or about 0.025% to 0.035%. In some embodiments, the nanoconjugate comprises about 0.03% of doxorubicin by weight of the nanoconjugate.

Nanoconjugates can be characterized by their hydrodynamic diameter (*i.e.,* hydrodynamic size). In some embodiments, the nanoconjugate disclosed herein has a hydrodynamic diameter of about 100 nm to about 150 nm or about 110 nm to about 140 nm as measured by dynamic light scattering (DLS). In some embodiments, the nanoconjugate has a hydrodynamic diameter of about 100 nm, about 110 nm, about 115 nm, about 120 nm, about 125 nm, about 130 nm, about 135 nm, about 140 nm, about 145 nm, or about 150 nm as measured by dynamic light scattering (DLS), *e.g.,* about 125 nm, or a range between any two of the preceeding values, e.g., about 120 nm to about 125 nm, about 120 nm to about 130 nm, or about 125 nm to about 130 nm. In some embodiments, the nanoconjugate has a hydrodynamic diameter of about 125 nm as measured by dynamic light scattering (DLS).

Nanoconjugates can also be characterized by their zeta potential. In some embodiments, the nanoconjugate disclosed herein has a zeta potential value of about -15 mV to about -30 mV. In some embodiments, the nanoconjugate has a zeta potential value of about -15 mV, about -16 mV, about -17 mV, about -18 mV, about -19 mV, about -20 mV, about -21 mV, about -22 mV, about -23 mV, about -24 mV, about -25 mV, about -26 mV, about -27 mV, about -28 mV, about -29 mV,or about -30 mV, e.g., about -23 mV, or a range between any two of the preceeding values, *e.g.*, about -15 mV to about -25 mV, about -20 mV to about -25 mV, or about -20 mV to about -30 mV. In some embodiments, the nanoconjugate has a zeta potential value of about about -20 mV to about -25 mV.

In one embodiment, the nanoconjugate disclosed herein consists essentially of a gold nanoparticle (AuNP), dithiolated diethylenetriamine pentaacetic acid (DTDTPA), a thioctic acid terminated bombesin peptide (TA-BBN), and doxorubicin, wherein the DTDTPA is directly linked to the gold nanoparticle surface via at least one Au-S bond, the TA-BBN is directly linked to the gold nanoparticle surface via at least one Au-S bond, and the doxorubicin is linked to the DTDTPA; wherein the AuNP-DTDTPA-BBN nanoconjugate precursor is prepared by a process comprising conjugating AuNP-DTDTPA nanoparticles with the TA-BBN, wherein the gold in the gold nanoparticle and the thioctic acid terminated bombesin peptide are present in the conjugation reaction in a stoichiometric ratio of about 1:2.

In certain embodiments, the nanoconjugate described above can comprse a gold nanoparticle (AuNP), dithiolated diethylenetriamine pentaacetic acid (DTDTPA), a thioctic acid terminated bombesin peptide (TA-BBN), and doxorubicin, wherein the nanoconjugate comprises about 15% to about 25% of TA-BBN, about 25% to about 35% of DTDTPA, and about 0.01% to about 0.05% of doxorubicin, by weight of the nanoconjugate.

In another embodiment, the nanoconjugate disclosed herein can comprise a gold nanoparticle (AuNP), dithiolated diethylenetriamine pentaacetic acid (DTDTPA), a thioctic acid terminated bombesin peptide (TA-BBN), and doxorubicin, wherein the nanoconjugate comprises about 20% of TA-BBN, about 30% of DTDTPA, and about 0.03% of doxorubicin, by weight of the nanoconjugate.

Although the targeting peptide is typically present on the nanoconjugates described herein, in some embodiments, the nanoconjugate disclosed herein does not comprise a thioctic acid terminated peptide. For example, in some embodiments, the nanoconjugate comprises a gold nanoparticle (AuNP), dithiolated diethylenetriamine pentaacetic acid (DTDTPA), and doxorubicin, wherein the DTDTPA is directly linked to the gold nanoparticle surface via at least one Au-S bond, and doxorubicin is linked to the DTDTPA as described elsewhere herein. This construct is referred to herein as AuNP(DTDTPA)-DOX.

In certain embodiments, the present disclosure provides a method for preparing the nanoconjugate disclosed herein, the method comprising coupling doxorubicin with a nanoparticle functionalized with DTDTPA. In some embodiments, the method comprises (1) conjugating AuNP-DTDTPA with a thioctic acid terminated peptide (e.g., TA-BBN) to form a conjugate; and (2) coupling the conjugate with doxorubicin to form the nanoconjugate.

In some embodiments, the coupling step forms an amide bond between a carboxyl group of DTDTPA and the amine group of DOX. In some embodiments, the coupling step takes place using an activating agent, such as EDC (1-Ethyl-3-(3-dimethylaminopropyl)carbodiimide)and sulfo-NHS (N-hydroxysulfosuccinimide).

In certain embodiments, the present disclosure provides a pharmaceutical composition comprising the nanoconjugate disclosed herein and a pharmaceutically acceptable carrier.

In certain embodiments, the present disclosure also provides compounds for use in a method for treating a cancer comprising administering a therapeutically effective amount of the nanoconjugate disclosed herein to a subject in need thereof. In some embodiments, the subject is a human.

In some embodiments, the cancer treated can be selected from the group consisting of acute lymphoblastic leukemia (ALL), acute myeloblastic leukemia (AML), bone sarcoma, breast cancer, endometrial cancer, gastric cancer, head and neck cancer, Hodgkin lymphoma, Non-Hodgkin lymphoma, liver cancer, kidney cancer, multiple myeloma, neuroblastoma, ovarian cancer, lung cancer (*e*.*g*., small cell lung cancer and non-small cell lung cancer), soft tissue sarcoma, thyomas, thyroid cancer, bladder cancer (*e*.*g*., transitional cell bladder cancer), uterine sarcoma, prostate cancer, colon cancer, ovarian cancer, pancreatic cancer, Wilms' tumor, and Waldenstrom macroglobulinemia.

In some embodiments, the cancer treated according to the methods described herein is associated with GRP overexpression. In some embodiments, the the cancer associated with GRP overexpression can be small cell lung cancer, prostate cancer, breast cancer, colon cancer, ovarian cancer, non-small cell lung cancer, or pancreatic cancer.

The nanoconjugate or pharmaceutical composition disclosed herein can be administered to a subject by various methods known to a person of ordinary skill in the art. For instance, the nanoconjugate or pharmaceutical composition of the present disclosure can be administered by injection or via oral administration.

The following examples are merely illustrative of the compositions and methods disclosed herein and are not intended to limit the scope hereof.

### EXAMPLES

General Methods: The materials used for synthesis of gold nanoparticle (AuNPs) were procured from standard vendors. Tetrachloroauric acid trihydrate (HAuCl₄.3H₂O), sodium borohydride (NaBH₄), diethylenetriaminepentacetic acid (DTPA), acetic anhydride, anhydrous pyridine, 2-aminoethanethiol hydrochloride, triethylamine, glacial acetic acid (CH₃COOH), Gallium nitrate (Ga(NO₃)₃), sodium hydroxide (NaOH), hydrocloric acid (HCl), methanol (MeOH), diethyl ether (Et₂O), sodium chloride (NaCl), dimethyl formamide (DMF), and dimethyl sulfoxide (DMSO), were purchased from Aldrich and used as received. For the preparation of aqueous solutions and for rinsing of gold nanoparticles, Milli-Q (DI) water (ρ>18MΩ) was used. MTT Cell Proliferation Assay kit was obtained from Promega Corporation, USA.

UV-Visible Spectroscopy: UV-Visible absorption spectra were recorded at room temperature using a Cytation 3 spectrophotometer (Biotek) in disposable 96 well plate with path length correction.

Electron Microscopy: Transmission electron microscope images were obtained on a JEOL 1400 transmission electron microscope (TEM), JEOL LTD., Tokyo, Japan. TEM samples were prepared by placing 5 µL of gold nanoparticle solution on the 300 mesh carbon coated copper grid. Excess solution was removed carefully and the grid was allowed to dry an additional five minutes. The average size and size distribution of nanoparticles were determined by processing the TEM image Adobe Photoshop with Fovea plug-ins.

Dynamic Light Scattering (DLS) and Zeta Potential Analysis: DLS measurements were performed with a Malvern Zetasizer Nano ZS (Malvern Instruments Ltd. USA) equipped with a 633-nm He-Ne laser and operating at an angle of 173°. The software used to collect and analyze the data was the Dispersion Technology Software (DTS) version 5.10 from Malvern. 600 µl of each sample was measured in low volume semi-micro disposable sizing cuvettes (Fisher Scientific, USA) with a path length of 10 mm. Triplicate measurements were made at a position of 4.65 mm from the cuvette wall with an automatic attenuator. For each sample, 15 runs of 10 seconds were performed. The size distribution, the Z-average diameter and the polydispersity index (PDI) were obtained from the autocorrelation function using the "general purpose mode" for all nanoparticle samples. The default filter factor of 50% and the default lower threshold of 0.05 and upper threshold of 0.01 were used. Zeta potential measurements were performed in triplicates using water as dispersant and Huckel model. For each sample, 20 runs were performed with auto analysis mode.

Nanoparticle Tracking Analysis: The hydrodynamic diameters of AuNPs were measured using NanoSight LM10-HSGFT system configured with a temperature controlled LM14G sample viewing unit equipped with a 532 nm (green) laser (NanoSight Limited, Amesbury, UK). Video tracking of the AuNPs based on Raleigh scattering was captured with a monochrome Marlin CCD camera (Allied Vision Technologies, Germany). A 1 mL syringe (Becton Dickinson, NJ) was used to deliver the samples to the viewing chamber and the temperature was held constant at 22°C. NanoSight 2.2 program was used to collect and analyze sample data. Each size measurement was based on a 30 second video and the Stokes-Einstein equation was used to calculate the mean hydrodynamic diameter. As noted below, the samples were diluted 30-fold relative to the stock AuNP concentration prior to NTA measurements. This dilution was selected such that ~900 particles were tracked in a 30 second video. These conditions provided a representative sampling of the entire sample and are confirmed by the fact that size distribution did not change with longer videos in which significantly more nanoparticles were analyzed. Three measurements were conducted for each sample to provide an average size and standard deviation.

Cell culture: PC-3 human prostate and MDA-MB231 human breast carcinoma cells (ATCC, USA) were grown in RPMI 1640 medium (obtained from Gibco BRL, Grand Island, NY) supplemented with 4.5 g/L D-glucose, 25 mM Hepes, 0.11 g/L sodium pyruvate, 1.5 g/L sodiumbicarbonate, 2 mM L-glutamine, 10% heat-inactivated fetal bovine serum (Atlanta Biologicals, USA) and 1% penicillin/ streptomycin antibitiotic solution. Cells were cultured in a humidified atmosphere of 95% air and 5% CO₂ at 37°C (Thermo Scientific, USA), with the medium changed every other day. The cells were adherent in monolayers and when confluent were harvested from the cell culture flasks with trypsin-EDTA (Invitrogen) and seeded further apart.

IC₅₀ measurements: The receptor binding affinity of conjugates was determined by a competitive cell-binding assay on PC-3 cell cultures using ¹²⁵I-Tyr4-bombesin as the GRP specific radio-ligand. Approximately 30,000 cells were incubated at 37°C for 40 minutes under 5% CO₂ in the presence of 20000 cpm ¹²⁵I-Tyr4-bombesin (2200 Ci/mmol) and increasing concentration of theconjugate tested. After incubation, the reaction medium was aspirated, and the cells were washed three times with cold RPMI 1640 modified buffer. Cell-associated radioactivity was determined by counting in a Packard Riastar γ counting system. IC₅₀ values were calculated using Graph Fit 4.0 graphing software. The % ¹²⁵I-Tyr4-bombesin bound to cells was plotted against increasing concentration of the conjugate tested to determine its IC₅₀ value.

In vitro Cytotoxicity Assay: In vitro cytotoxicity evaluation of NP1-DOX and NP2-DOX with respective controls such as NP1, NP2, free doxorubicin was performed on GRP expressing and non-expressing cancer cells, PC3 and MDA-MB-231, respectively. The cytotoxicity evaluation was performed in triplicates and the protocol was followed as described by the manufacturer. Briefly, 1 × 10⁵ ml⁻¹ cells at the exponential growth phase were placed in a flat bottom 96-well polystyrene-coated plate and were incubated for 12 hour in a CO₂ incubator at 5% CO₂ and 37 °C. A series of concentrations ranging from 0 to 10 ug/mL (0, 0.31, 0.625, 1.25, 2.5, 5.0 and 10 µg/mL) of NP1-Dox and NP2-Dox was prepared in the medium. Each concentration was added to the plate in quadruplets. After 24 hour incubation, 10 µL per well MTT (as received from the manufacturer, Promega Corporation, USA) was added and kept for 4 hours, and the formazan crystals so formed were dissolved in 100 µL detergent/solubilizing buffer. The plates were kept for 2 hours in dark at 25°C to dissolve all crystals, and the intensity of developed color was measured by micro plate reader (Epoch, BioTek, USA) operating at 570 nm wavelength. Wells with complete medium, nanoparticles, and MTT, but without cells, were used as blanks. Untreated cells were considered 100% viable.

In Vitro Stability studies: In vitro stability studies were performed by incubating solutions of NP1 and NP2 at various pH conditions: 2, 5, 7, 10 and 12 for the period of 24 hours. The stability behavior for both were also monitored by challenging aqueous solutions of NP1 and NP2 (0.5 mL) with 0.5 mL each of 0.2M cysteine, 0.2M histidine, 0.2M HSA and 10% saline solutions. The stability was measured by monitoring the UV-visible absorbance, hydrodynamic radius and zeta potential measurements at 0 hour to 96 hours (0, 1, 24, 48, 72, and 96 hours). A negligible change in UV-Vis plasmon band of NP1 and NP2 confirmed the retention of nanoparticulate composition with stable behavior in all the challenging solutions except cysteine. The treated solutions did not show any noticeable change in hydrodynamic radii, thus confirming the stability of these conjugates.

### Example 1

### Synthesis and Characterization of BBN-AuNP-DTDTPA Conjugates

Dithiolated diethylenetriaminepentaacetic acid (DTDTPA) functionalized gold nanoparticles (AuNP-DTDTPA; **NP1**) were used as a precursor to conjugate the targeting peptide, bombesin (BBN), and a chemotherapeutic agent, DOX, on the surface of the nanoparticle. *See* **Figure 1**.

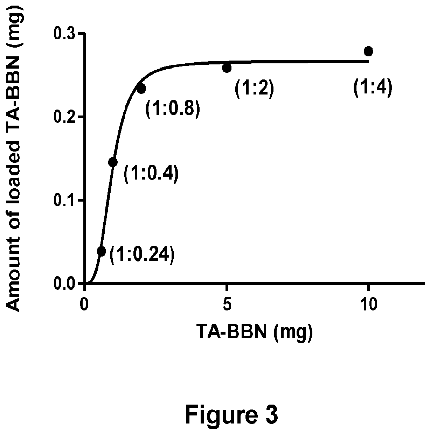

The first step was to conjugate the targeting peptide to **NP1**. Thioctyl terminated bombesin (TA-BBN) was used for conjugating the peptide to **NP1** through gold-thiol bonding. Three different stoichiometric ratios of AuNP:BBN were used (1:0.5, 1:2 and 1:4). Excess of unreacted BBN was removed by repeated washings and BBN-AuNP-DTDTPA (**NP2**) was isolated as pure nanoparticle pellets. HPLC analysis of the supernatant solutions obtained after conjugation of TA-BBN to **NP1** was utilized for quantifying TA-BBN bound to **NP2**. A standard calibration curve was constructed using different concentrations of TA-BBN and the area under curve (AUC) of known concentrations of TA-BBN used in the reactions were determined by HPLC. The analysis of AUC in the supernatants and known concentration of TA-BBN were correlated to determine the amount of TA-BBN conjugated on the surface of **NP2** (**Figure 3**). Conjugating the AuDTDTPA construct with TA-BBN in a 1:2 ratio (Au:TA-BB) resulted in about 0.26 mg of TA-BBN being incorporated into the construct per 1 mg of AuDTDTPA. *See* **Figure 3****. NP2** prepared using 1:2 ratio showed remarkable stability and was therefore chosen for further studies.

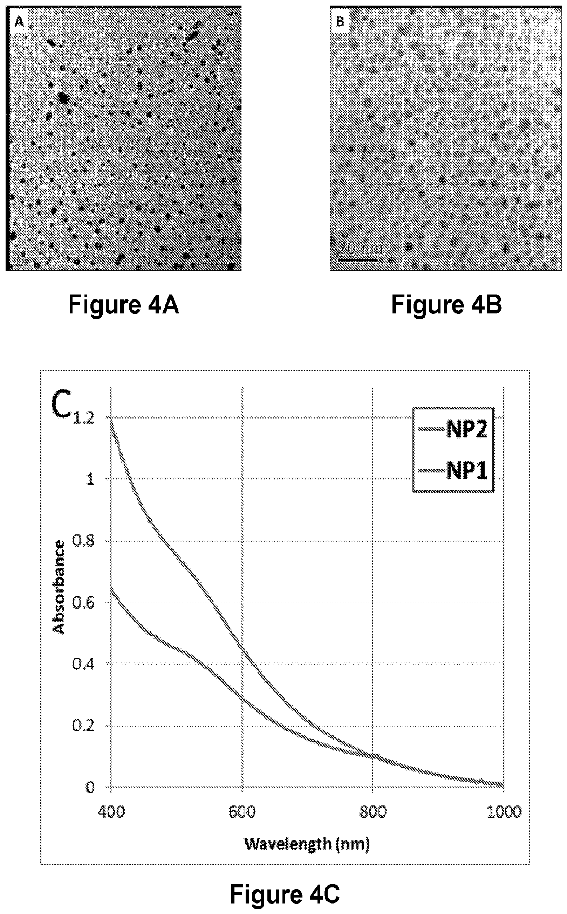

Nanoconjugates were characterized in detail by transmission electron microscope (TEM) image analysis, UV-visible absorption spectroscopy, dynamic light scattering (DLS), and Zeta potential measurements. TEM images showed a uniform size distribution of the nanoconjugates, with a size range of 3-5 nm (**Figures 4A** and **4B**). **NP2** showed no change in UV-visible absorption spectrum compared to **NP1** upon conjugation to BBN. (**Figure 4C**). A 20 nm change in hydrodynamic diameter was observed in **NP2** compared to **NP1** upon conjugation to BBN (1:2, Au:BBN). The zeta potential change was negligible (~3 units) in **NP2** compared to **NP1**. The average particle size determined using nanoparticle tracking analysis (NTA) correlated well with the dynamic light scattering measurements. The physicochemical properties of **NP1** and **NP2** conjugates are summarized in **Table 1**.

**Table 1**

| | **DLS hydrodynamic Size (*d* nm)** | **Charge (mV)** |
|---|---|---|
| **NP1** | 78 | -72 |
| **NP1-DOX** | 95 | -23 |
| **NP2** | 105 | -69 |
| **NP2-DOX** | 124 | -23 |

### Example 2

### XPS Analysis

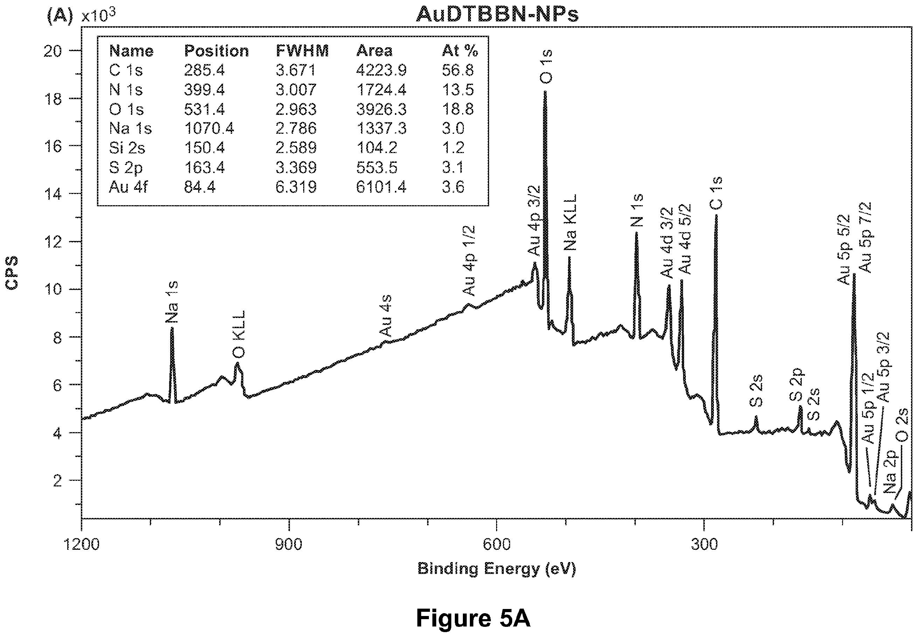

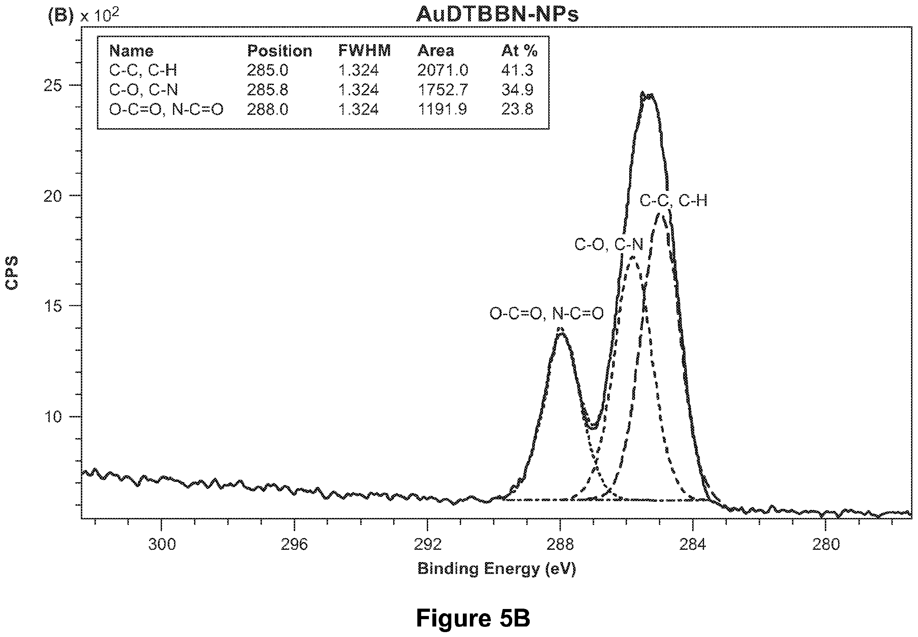

To understand the nature of bonding and chemical interactions between AuNP-DTDTPA and TA-BBN, detailed XPS analysis was performed. The XPS spectrum of **NP2** exhibits characterisitc peak of C, N, O, S, and Au (**Figure 5A**)**.** The XPS high-resolution spectrum of the C1s regions shows various C-C, C-H, C-O, C-N, O-C=O, N-C=O regions as expected from conjugated TA-BBN peptide (**Figure 5B**). The relative elemental compositions and peak assignments for carbon species of **NP2** are shown in the **Tables 2** and **3**, respectively.

**Table 2: Relative Elemental Compositions of BBN-[AuNP(DTDTPA)] as Determined by XPS [Atom %]**

| | C | N | O | Na† | Si^{∗} | S | Au |
|---|---|---|---|---|---|---|---|
| BBN-[AuNP(DTDTPA)] | 57 | 14 | 19 | 3.0 | 1.2 | 3.1 | 3.6 |

| | | | | | | | |
|---|---|---|---|---|---|---|---|
| † Sodium detected due to dissolution in NaOH solution ^{∗} Silicon detected due to deposition on silicon wafer | | | | | | | |

**Table 3: Relative Compositions and Most Probable Peak Assignments for Carbon Species as Determined by XPS, C1s Region [Atom %]**

| | C-C, C-H | C-O, C-N | O-C=O, N-C=O |
|---|---|---|---|
| BBN-[AuNP(DTDTPA)] | 41 | 35 | 24 |

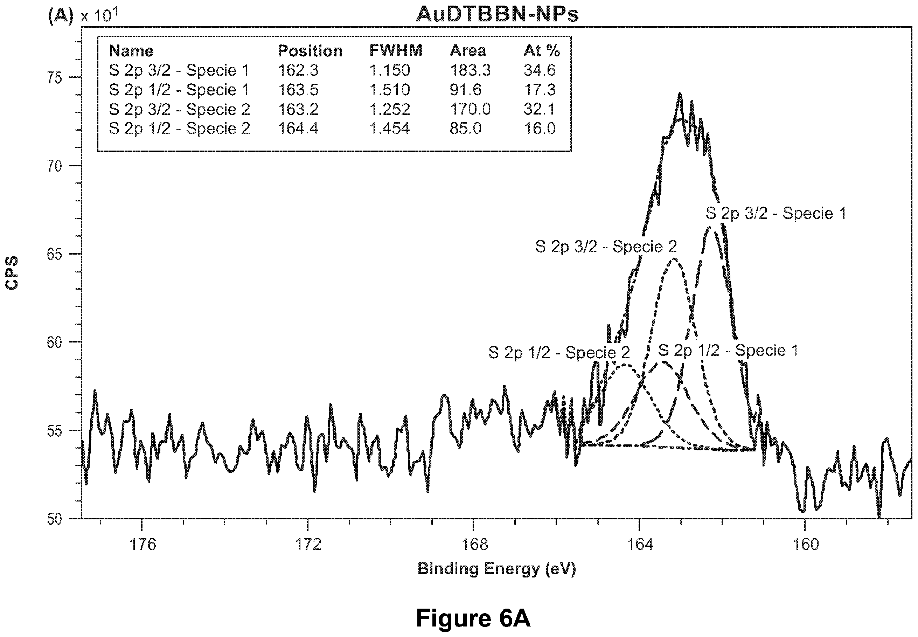

Analysis of the XPS hi-resolution spectrum for the S 2p region suggests that there are two different S species (Species 1 and Species 1) and each is split in to a doublet. Species 1 exhibits two distinct sulfur peaks at 162.3 and 163.5eV, attributed to Au-S bonding of DTDTPA. The doublet sulfur peak, S 2p_{3/2} and S 2p_{1/2} with coupling of 1.2 ev, confirms the adsorption of thiols on gold surface. The binding energy and coupling values correlate well with those reported in literature for **NP1**; however, the peaks shift to a slightly higher binding energy (0.5 eV) region upon conjugation to BBN. Additionally, the two distinct sulfur peaks for Species 2 at 163.2 and 164.4eV with a ratio of 2:1 correspond to Au-S (sulfur coordinated to AuNPs) and S-CH3 (sulfur present in the methylcysteine amino acid in the BBN peptide backbone), respectively. Each class of sulfur peak was further split into the doublet S2p3/2 and S2p1/2 with coupling of 1.2 eV, confirming the conjugation of BBN to AuNP as reported in literature. Such presence of doublet with an equivalent area at a higher binding energy indicates both sulfur atoms of TA-BBN formed an Au-S bond on the AuNP surface. Upon conjugation with BBN, the binding energy values shift to a lower energy region compared to AuNP-BBN reported in literature. This may be due to the difference in the core size of the nanoparticles that were used for conjugation of BBN. The energies for elemental S, and some organic forms of S fall in the range of 162-164 eV (**Figure 6A****)**. Because no further peaks are observed in the higher energy region of the spectrum corresponding to oxidized sulfur, these measurements are consistent with the observation that the BBN-AuNP-DTDTPA (**NP2**) is the exclusive product formed during the nanoconjugation reaction.

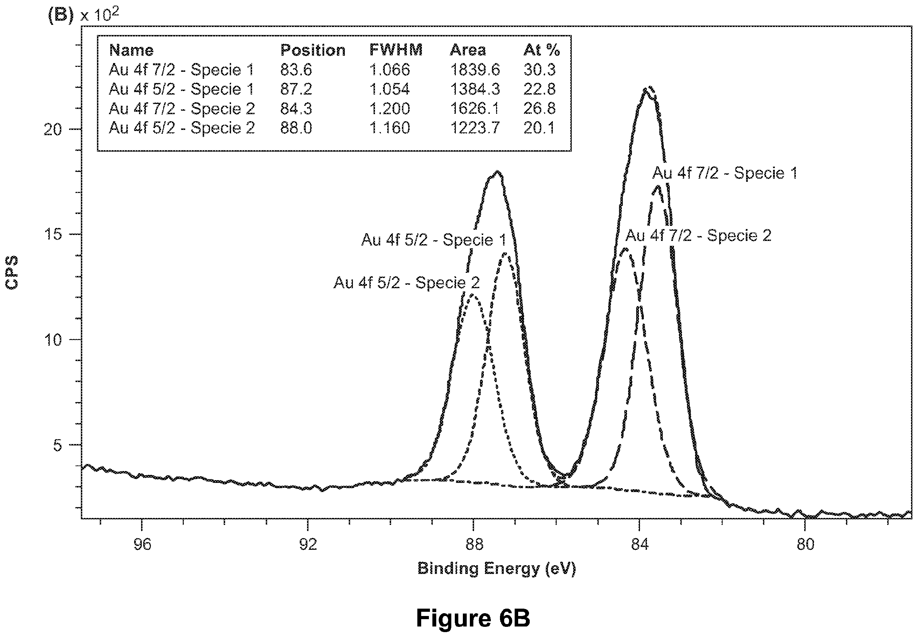

The 4f region of the spectrum shows two different signals for gold with energies of 83.6, 84.3, and 87.2, 88.0eV for Au 4f_{7/2} and Au 4f_{5/2}, respectively, corresponding to the core gold atom Au (0) and surface gold with partial Au (I) character (**Figure 6B**). It is widely believed from various recent studies that disulfide in thioctic acid oxidatively adds to AuNPs, resulting in a partial Au (I) character at the surface of the nanoparticle. The signal corresponding to the binding energies of 84.3 and 88 eV for Au peaks in 4f region are similar to the peptide conjugated AuNPs reported in literature, suggesting that Au is bound to the S atom of DTDTPA and TA-BBN. Consistent with this fact, two different oxidation states of gold corresponding to surface and core gold atoms were noted in the XPS spectrum. Results from various XPS studies on gold nanoparticles confirmed similar findings. The above observations clearly establish the binding of TA-BBN in **NP2** nanoconjugate.

### Example 3

### Synthesis and Characterization of BBN-AuNP-DTDTPA-DOX Conjugates

The carboxyl groups of DTDTPA on the surface of AuNPs are available for further functionalization. These carboxyl groups were utilized for conjugating doxorubicin. Traditional EDC-sulfoNHS chemistry was used to conjugate the amine group of DOX to the carboxyl groups of DTDTPA to obtain non-targeted AuNP-DTDTPA-Doxorubicin (**NP1-DOX**) and targeted BBN-AuNP-DTDTPA-DOX (**NP2-DOX**).

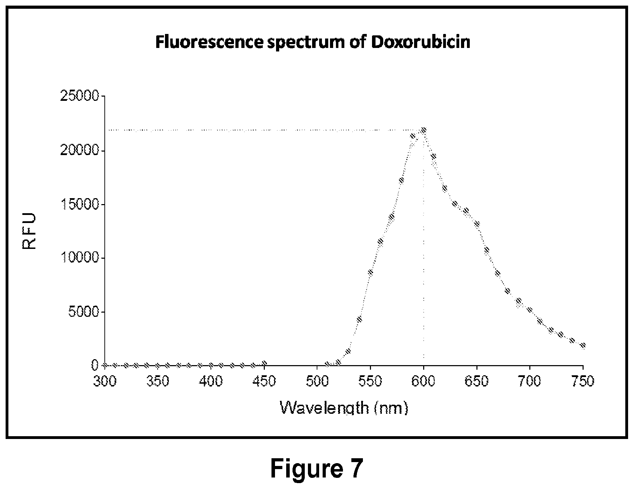

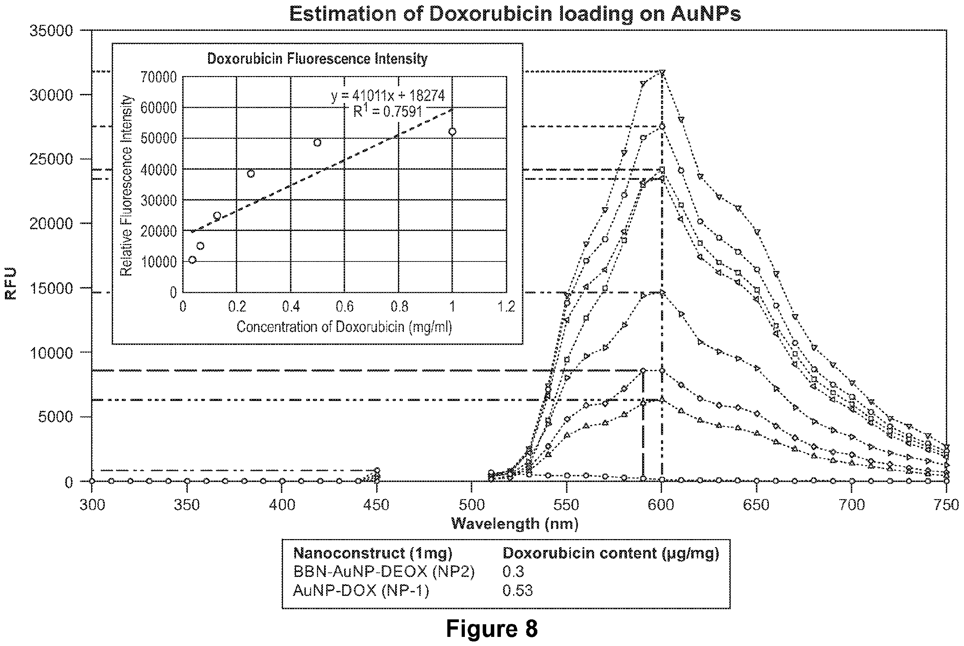

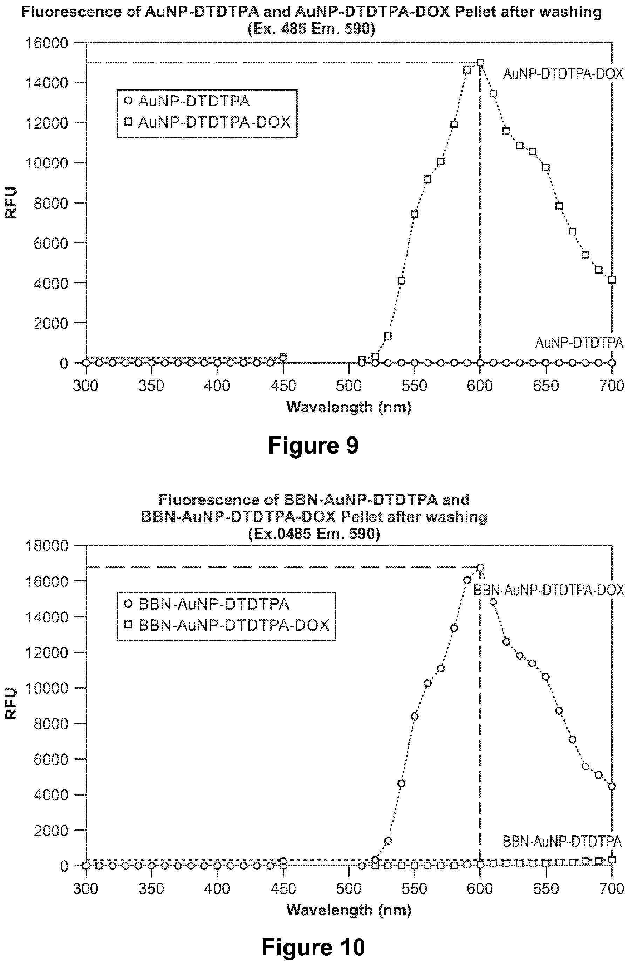

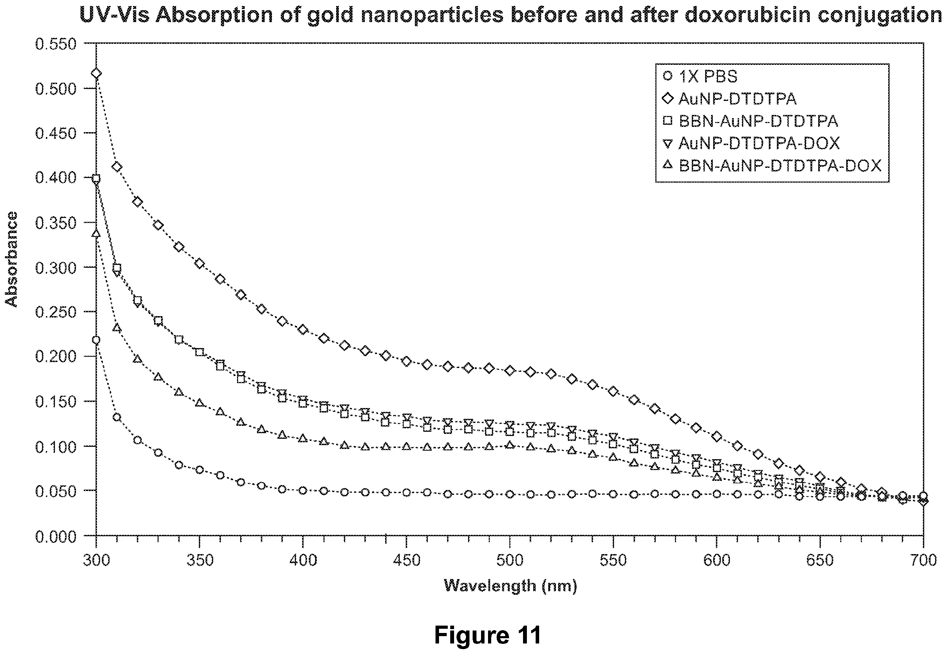

Doxorubicin loading on **NP1** and **NP2** was estimated by fluorescence spectroscopy. Doxorubicin shows a fluorescence signature at 600 nm when excited at 485 nm and emitted at 590 nm (**Figure 7**). Serial dilutions of DOX were made and a standard calibration curve was constructed to record the fluorescence intensities (**Figure 8**). The fluorescent intensities of the supernatants were recorded and correlated with the standard calibration curve to quantify the amount of doxorubicin conjugated to AuNPs. Through fluorescent measurements, it was found that 0.53 µg and 0.3 µg of doxorubicin per mg of AuNP-DTDTPA and BBN-AuNP-DTDTPA were conjugated, respectively. Additionally, serial dilutions of **NP1-DOX** and **NP2-DOX** were made and the fluorescent intensities were recorded. The fluorescence intensity variation was proportional and consistent, confirming the conjugation of DOX to **NP1** and **NP2**. The fluorescence measurement also suggests that doxorubicin fluorescence remains unaffected upon conjugation on AuNPs (**Figure 9** and **10**). The UV-visible spectra of **NP1-DOX, NP2-DOX** with their respective controls is showed in **Figures 11****.** A 10-20 nm change in hydrodynamic diameter was observed upon conjugation with DOX with both **NP1** and **NP2**. The zeta potential shifted to positive upon conjugation with DOX. The negative zeta potential value of -72 and -69 mV for **NP1** and **NP2**, respectively, changed to -23 mV indicates that the particles repel each other and that there is no tendency for the particles to aggregate. The physicochemical properties of **NP1-DOX** and **NP2-DOX** conjugates are summarized in **Table 1**.

### Example 4

### In Vitro Stability Studies

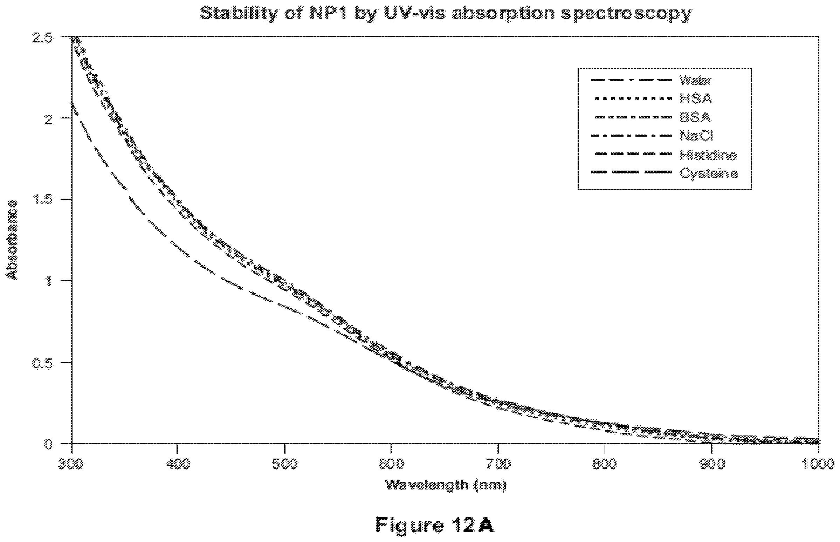

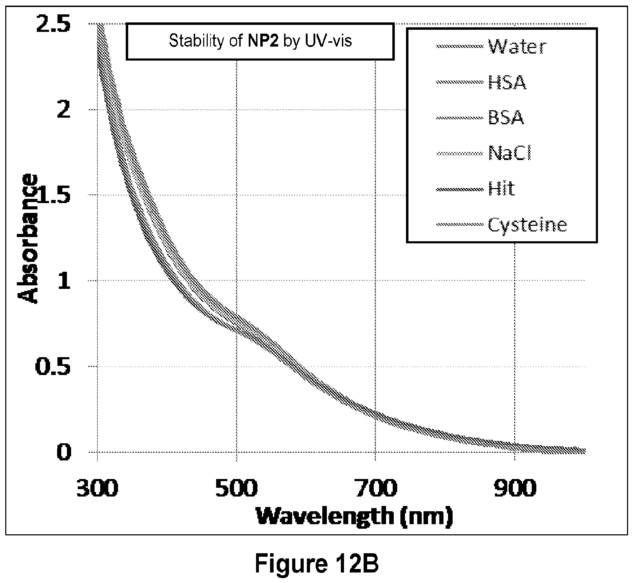

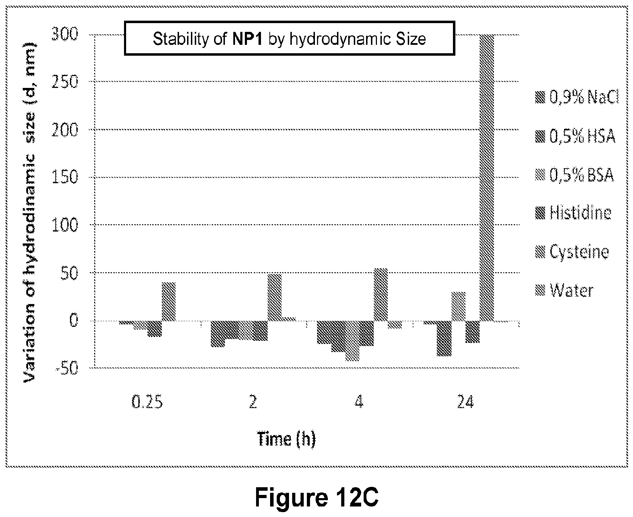

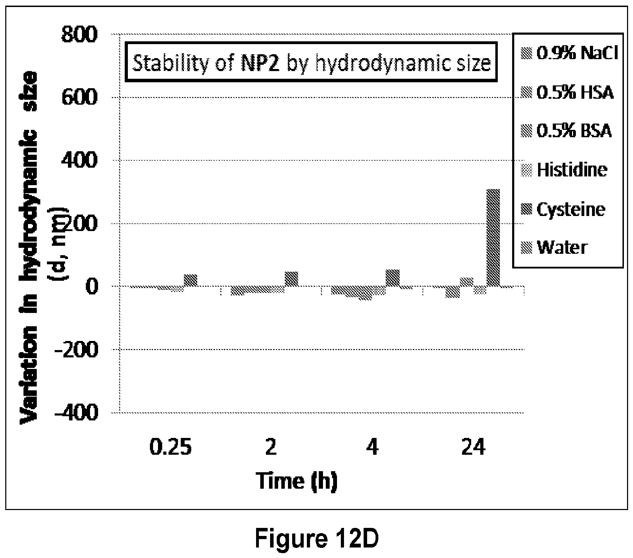

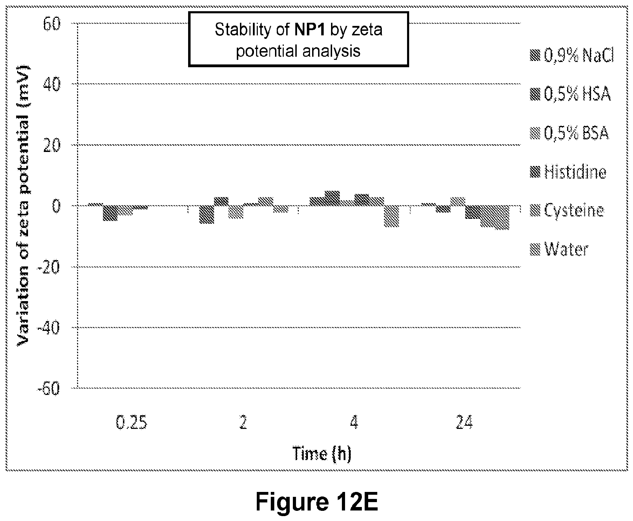

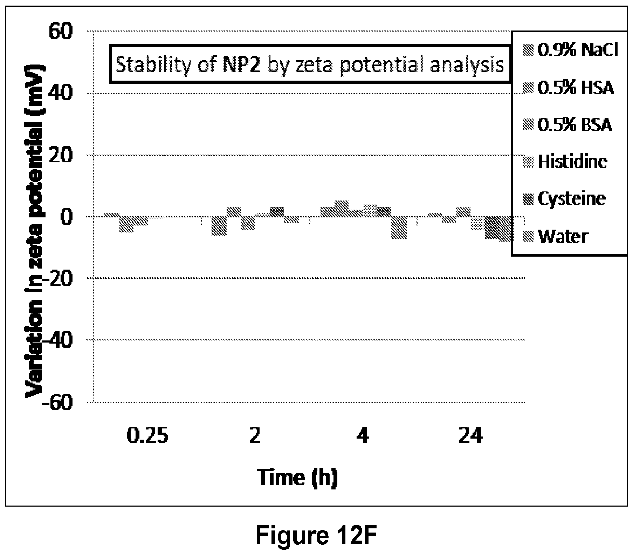

The stability of **NP1** and **NP2** conjugates was investigated in various biologically relevant solutions. Solutions of **NP1** and **NP2** were challenged with 0.9% NaCl, 0.5% cysteine, 0.2M histidine, 0.5% human serum albumin (HSA), and 0.5% bovine serum albumin (BSA). The stability of the nanoconjugates at different time points was analyzed by three techniques, (i) monitoring the plasmon resonance using UV-visible spectroscopy, (ii) Changes in hydrodynamic diameter, and (iii) electrophoretic charge (Zeta potential) measurements. The change in surface plasmon resonance wavelength and plasmon band width and measurement of hydrodynamic diameter is directly related in understanding the aggregation behavior of the nanoparticles. Zeta potential measurement is an indication of repulsive forces that are present in aqueous solution and can be used to predict the long-term in vitro/in vivo stability of nanoparticulate dispersions. The stability studies were performed for nanoconjugates prepared with 1:2 and 1:4 stichiometric ratios of Au:BBN. The use of 1:4 ratio of Au:BBN showed aggregation over the period of 24 hrs time while both conjugates **NP1** and **NP2** [1:2 (Au:BBN) ratio] were stable in these biologically relevant solutions. The UV-visible absorption peaks remain unaltered, the changes in hydrodynamic diameter and zeta potential are minimal confirming the structural integrity and robustness of the conjugate (**Figure 12**). However, in the presence of cysteine it was observed that both **NP1** and **NP2** showed increase in hydrodynamic size over a period of time (24 hr) due to gold thiol interactions and the increase was not significant. The *in vitro* stability data indicate that **NP1** and **NP2** have optimum kinetic stability for use in subsequent receptor-targeting disclosures *in vivo.*

### Example 5

### In Vitro receptor binding affinity studies of NP2

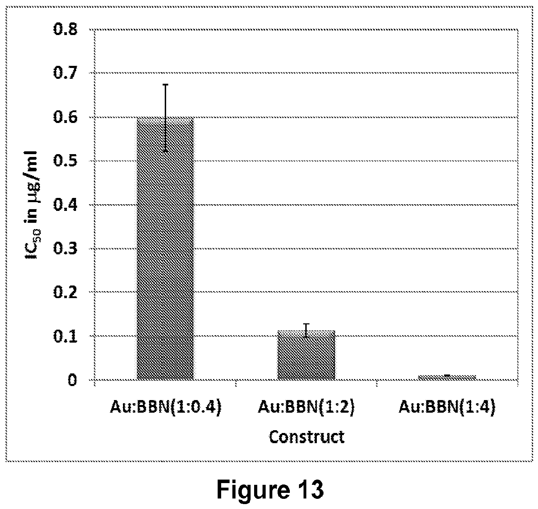

Human prostate tumor PC-3 cells express a large number of GRP receptors on the surface of the cells. The binding affinity of **NP2** was evaluated by performing competitive inhibition assay and determining the IC₅₀ values. Nanoparticles prepared with three different stoichiometric ratios of Au:BBN (1:0.5, 1:2, 1:4) were used. A comparison of IC₅₀ values for different stoichiometric ratios of Au:BBN (1:0.5, 1:2, 1:4) was plotted (**Figure 13**). The IC₅₀ values of different ratios of the conjugate **NP2** are reported in micrograms, as the molecular weights of the nanoconjugates cannot be accurately determined. It is evident from the data that the IC₅₀ values or cell binding affinity of conjugates depend on the degree of TA-BBN peptide coating over the surface of AuNPs. For instance, conjugate **NP2** with Au:BBN= 1:4 ratio, which has a greater number of TA-BBN peptides molecules on the surface of AuNPs, exhibited a lower IC₅₀ value (or higher cell-binding affinity) when compared with other conjugates. However, this conjugate is not highly stable and was not used for cellular studies. The competitive inhibition study confirms the receptor targeting properties of **NP2.**

### Example 6

### In vitro Cytotoxicity Assays:

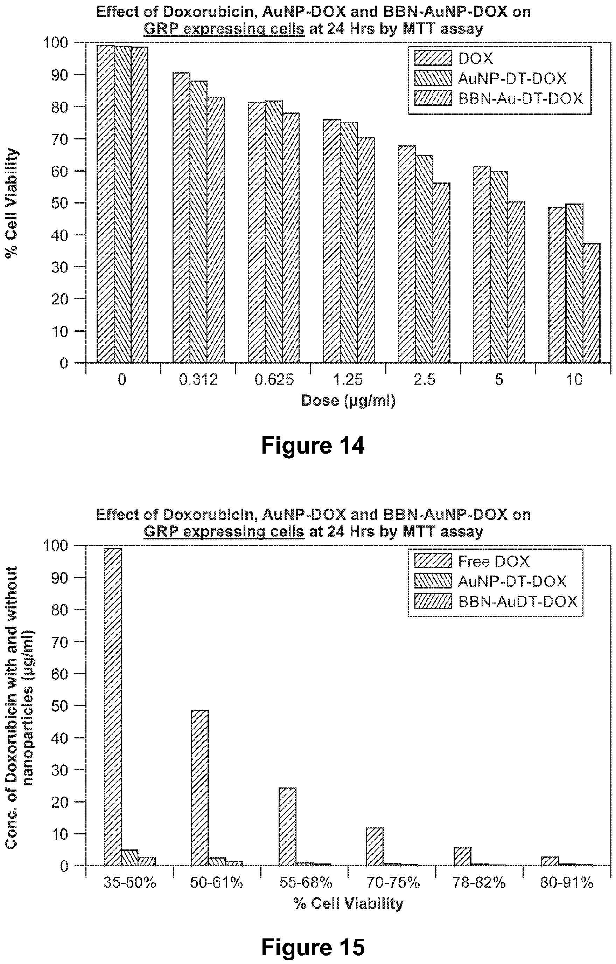

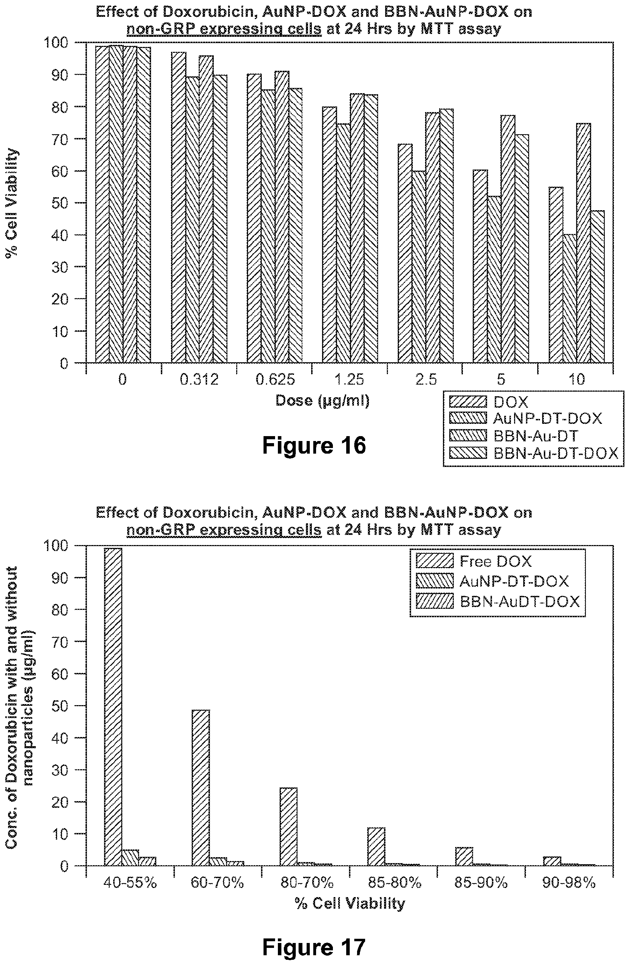

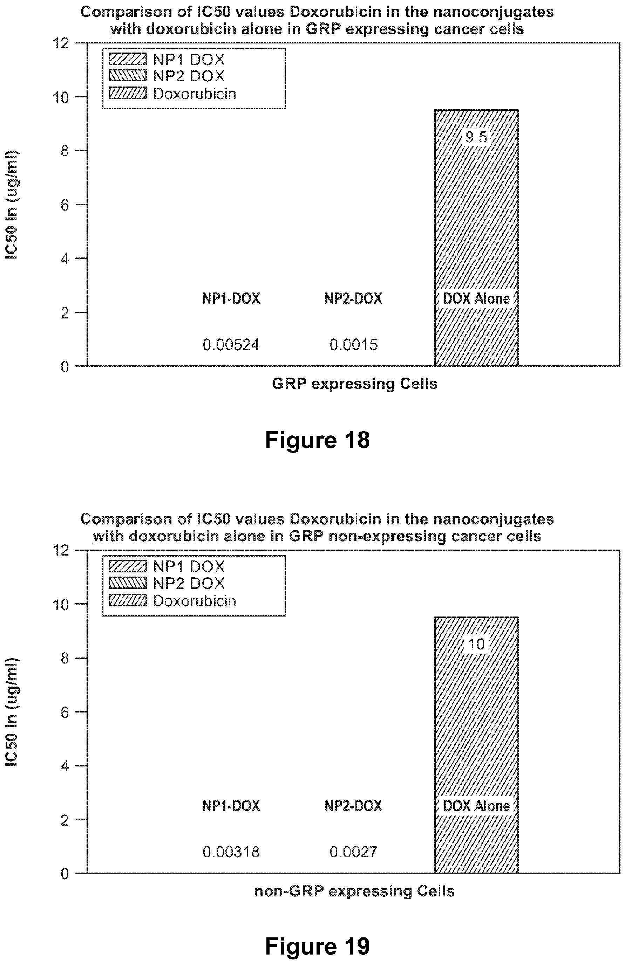

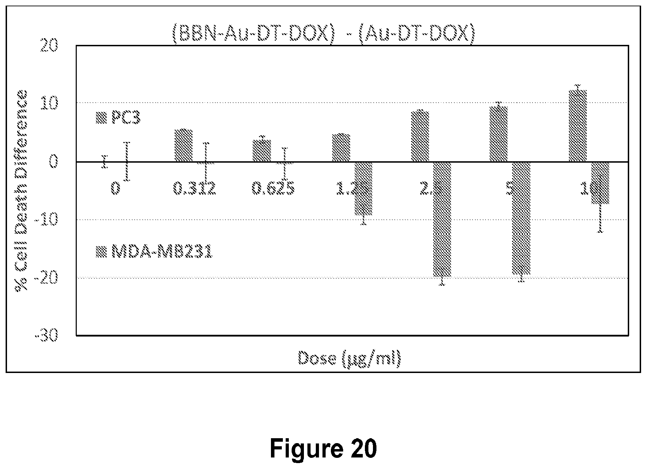

The *in vitro* cytotoxicity of doxorubicin conjugated AuNPs, NP1-DOX and NP2-DOX, on GRP expressing and non-GRP expressing human cancer cells was examined with proper controls by MTT assay. Human prostate cancer (PC3) cells have large number of GRP receptors on the surface of cells. Specifically, literature shows that 44000 bombesin receptor sites are present on the surface of human prostate cancer cells. Human breast (MDA-MB231) cancer cells were used as non-GRP expressing cancer cells. Free doxorubicin showed IC₅₀ of 9.5 µg/ml in PC3 (**Figure 14**) and >10 ug/ml in MDA-MB231 (**Figure 16**) cancer cells. A comparative chart showing the effect of **NP1-DOX** and **NP2-DOX** with DOX on the cytotoxicity of both GRP and non-GRP expressing cells is shown in **Figures 15****,** **17****, and** **20****. NP1-DOX** and **NP2-DOX** showed IC₅₀ of 6 µg/ml and 9 µg/ml, respectively, in non-GRP expressing cancer cells, while in PC3 cells the IC₅₀ values were found to be 10 µg/ml and 5 µg/ml, respectively (**Table 4**). It should be noted that the IC₅₀ values represented here for **NP1-DOX** and **NP2-DOX** is based on the total weight of the nanoconjugate and the amount of DOX loaded on the nanoconjugate is 0.53µg/mg and 0.3 µg/mg, respectively. The IC₅₀ values of the nanoconjugates in terms of doxorubicin are listed in Table 4 and **Figures 18** and **19**.

**Table 4**

| | IC₅₀ (µg/ml) | | | |
|---|---|---|---|---|
| | PC3 cells | | MDA-MB231 cells | |
| | Nanoconj ugate Conc. | Doxorubicin Conc. | Nanoconjugate Conc. | Doxorubicin Conc. |
| **NP1-DOX** | 9.89 | 0.0052 | 6 | 0.00318 |
| **NP2-DOX** | 5 | 0.0015 | 9 | 0.0027 |
| Doxorubicin | 9.5 | 9.5 | >10 | >10 |

As can be seen above, the doxorubicin conjugated AuNPs exhibited much more potent cytotoxicity than free doxorubicin. For example, NP2-DOX's cytotoxivity increased 3000-fold and 6000-fold as compared to free doxorubicin in non-GRP expression and GRP expression cancer cells, respectively.

### Example 7

### Synthesis of AuNP-DTDTPA

The dithiolated diethylenetriaminepentaacetic acid (DTDTPA) functionalized AuNPs were prepared via a modified protocol by Brust et. al., J. Chem. Soc., Chem. Commun. 1995, 1655-1656. Briefly, 200 mg (0.507 mmol) of HAuCl₄.3H₂O was dissolved in 120 ml of methanol in a 500 ml round bottom flask. In a separate flask, 482 mg (0.943 mmol) of DTDTPA was dissolved in 40 ml of MeOH and 2 ml of glacial acetic acid. This solution was added with continuous stirring to aqueous solution of gold salt to produce an orange solution. To this mixture, 190 mg (5 mmol) of NaBH₄ dissolved in 14 ml of DI water was added under vigorous stirring at room temperature. Immediately after addition of NaBH₄, the solution became dark brown followed by appearance of black flocculate. The resultant mixture was allowed to stir for 1 hour at room temperature and then 5 ml of 1M aqueous HCl was added. This black solution of gold nanoparticles was then subjected to centrifugation at 7000 RPM for 20 minutes. The supernatant was removed and the particles were washed twice with 0.01M HCl keeping the centrifugation parameters same as above. The particles were further washed thoroughly and successively with DI water followed by diethyl ether. The resulting black powder of AuNP-DTDTPA was dried under vacuum and stored at -20°C. As needed, the particles were readily dispersed in 0.01M NaOH and were stable for over a month.

### Example 8

### Synthesis of Thioctic Acid-BBN Peptide

The synthesis BBN was performed using solid phase peptide synthesis employing Fmoc chemistry methodology and the final peptides were purified by HPLC. A 4-hydroxymethylphenoxyacetyl- 4'-methylbenzyhydrylamine resin was used as the solid support for the synthesis. Fmoc-protected amino acids were activated using one equivalent of 0.45 M HBTU/HOBt solutions and two equivalents of N,N-diisopropylethylamine. The amino acids were Fmoc deprotected using piperidine and coupled using NMM.HBTU. Following the coupling of all of the amino acids in the appropriate sequence, thioctic acid (lipoic acid) was coupled using DIC.HOBt. Cleavage of the peptide from the resin was performed using TFA. This cleavage step also removed the amino acid side chain protecting groups. The peptide was purified on a reverse-phase HPLC/C18 column using an AB gradient from 0% B, where A is 0.1% TFA in water and B is 0.1% TFA in acetonitrile. Following purification, the peptide mass was measured by liquid chromatography-mass spectrometry or matrix-assisted laser desorption/ionization, and the purity was measured using HPLC reverse-phase chromatography.

### Example 9

### Synthesis of bombesin receptor specific gold nanoconjugates stabilized with DTDTPA (BBN-AuNP-DTDTPA)

Thioctic acid terminated bombesin was reacted with gold nanoparticles with stiochiometric ratios of Au:BBN 1:0.25, 1:0.5, 1:1, 1:2 and 1:4. Typically, In a 20 ml glass vial, a solution of AuNP-DTDTPA ([Au]=2.28 µmol) using aqueous/methanolic mixture (1:9) of 0.01M NaOH was prepared. Thioctic acid terminated bombesin (TA-BBN) 0.64 mg (0.57 µmol), 1.28 mg (1.14 µmol), 2.56 mg (2.27 µmol), 5.12 mg (4.54 µmol) and 10.24 mg (9.08 µmol) were dissolved in 4 mL of MeOH and then added to the nanoparticles solution. The reaction mixture was stirred for 2 hours at room temperature and formation of a dark brown precipitate was observed. The mixture was centrifuged (9300 g for 10 min at 20°C) and the supernatant was removed. The precipitated AuNPs were washed two times with MeOH and three times with water. The BBN-AuNP-DTDTPA were dried at low pressure and stored at -20°C.

### Example 10

### Synthesis of AuNP-DTDTPA-Doxorubicin and BBN-AuNP-DTDTPA-Doxorubicin

Synthesis of doxorubicin conjugated gold nanoparticles with and without bombesin peptide was performed in triplicates. The gold nanoparticles, 0.5 mg were suspended in IX PBS by sonication. To 100 µl of this solution, 2 mg of 1-Ethyl-3-[3-dimethylaminopropyl]carbodiimide hydrochloride (EDC) and 2 mg of Sulfo-NHS (N-hydroxysulfosuccinimide) in 0.1 M 2-(morpholino)ethane sulfonic acid (MES) buffer (pH 4.6) was added. The pH of the reaction mixture was maintained at 5.3-5.4 using MES buffer. The reaction was stirred for 3hrs at 37⁰C at 700 RPM. After 3 hours, the reaction mixture was centrifuged at 20000 g for 20 min at 25°C. Clinical doxorubicin 0.7 mg was used for conjugation with nanoparticles. To the doxorubicin solution, nanoparticles with activated carboxyl groups were added and the reaction mixture was vortexed. The coupling was performed for overnight at 25°C at 750 rpm. Reaction mixture was centrifuged at 20000 g for 20 minutes at 25°C and the pellets wwere subsequently washed twice with IX PBS and resuspended in IX PBS solution. Both the pellets and supernatants were used to study conjugation efficiency by fluorescence spectroscopy analysis.

## Claims

1. A nanoconjugate comprising: a gold nanoparticle (AuNP), dithiolated diethylenetriamine pentaacetic acid (DTDTPA), and doxorubicin,
wherein the DTDTPA is directly linked to the gold nanoparticle surface via at least one Au-S bond; and
the doxorubicin is linked to the DTDTPA.

2. The nanoconjugate of claim 1, further comprising a thioctic acid terminated peptide directly linked to the gold nanoparticle surface via at least one Au-S bond, and, optionally,
wherein the thioctic acid terminated peptide is thioctic acid terminated bombesin.

3. The nanoconjugate of claim 2, wherein the thioctic acid terminated bombesin has a sequence: Lipoic-Gln-Trp-Ala-Val-Gly-His-Leu- Met-NH₂.

4. The nanoconjugate of any one of claims 1 to 3, wherein the doxorubicin is linked to the DTDTPA via an amide bond.

5. The nanoconjugate of any one of claims 2 to 4, which is prepared by a process
comprising conjugating AuNP-DTDTPA nanoparticle with the thioctic acid terminated peptide, wherein the gold in the gold nanoparticle and the thioctic acid terminated peptide are present in the conjugation reaction in a stoichiometric ratio of about 1:0.5 to about 1:4.

6. The nanoconjugate of claim 5, wherein the gold in the gold nanoparticle and the thioctic acid terminated peptide are present in the conjugation reaction in a stoichiometric ratio of about 1:2.

7. The nanoconjugate of any one of claims 2 to 6, wherein the thioctic acid terminated peptide comprises about 1% to about 40% by weight of the nanoconjugate.

8. The nanoconjugate of claim 7, wherein the thioctic acid terminated peptide comprises about 20% to about 30% by weight of the nanoconjugate.

9. The nanoconjugate of any one of claims 1 to 8, wherein the doxorubicin comprises about 0.01% to about 0.05% by weight of the nanoconjugate.

10. The nanoconjugate of claim 9, wherein the doxorubicin comprises about 0.03% by weight of the nanoconjugate.

11. The nanoconjugate of any one of claims 1 to 10, wherein the nanoconjugate has a hydrodynamic diameter of about 110 nm to about 140 nm as measured by dynamic light scattering (DLS).

12. The nanoconjugate of claim 11, wherein the nanoconjugate has a hydrodynamic diameter of about 120 nm to about 130 nm as measured by dynamic light scattering (DLS).

13. The nanoconjugate of any one of claims 1 to 12, wherein the nanoconjugate has a zeta potential value of about -15 mV to about -30 mV.

14. The nanoconjugate of claim 13, wherein the nanoconjugate has a zeta potential value of about -20 mV to about -25 mV.

15. The nanoconjugate according to claim 1, comprising: a thioctic acid terminated peptide, wherein the thioctic acid terminated peptide is directly linked to the gold nanoparticle surface via at least one Au-S bond.

16. A pharmaceutical composition comprising the nanoconjugate of any one of claims 1 to 15, and a pharmaceutically acceptable carrier.

17. The nanoconjugate of any one of claims 1 to 15 for use in treating a cancer comprising administering a therapeutically effective amount of the nanoconjugate to a subject in need thereof.

18. The nanoconjugate of claim 17 for use in treating a cancer, wherein the cancer is selected from the group consisting of acute lymphoblastic leukemia (ALL), acute myeloblastic leukemia (AML), bone sarcoma, breast cancer, endometrial cancer, gastric cancer, head and neck cancer, Hodgkin lymphoma, Non-Hodgkin lymphoma, liver cancer, kidney cancer, multiple myeloma, neuroblastoma, ovarian cancer, lung cancer, soft tissue sarcoma, thyomas, thyroid cancer, bladder cancer, uterine sarcoma, prostate cancer, colon cancer, ovarian cancer, non-small cell lung cancer, pancreatic cancer, Wilms' tumor, and Waldenstrom macroglobulinemia.

19. The nanoconjugate of claim 17 or claim 18 for use in treating a cancer by injection.

## Patentansprüche

1. Nanokonjugat, umfassend: einen Goldnanopartikel (AuNP), dithiolierte Diethylentriaminpentaessigsäure (DTDTPA) und Doxorubicin, wobei das DTDTPA über mindestens eine Au-S-Bindung direkt an die Oberfläche des Goldnanopartikels gebunden ist; und wobei das Doxorubicin an das DTDTPA gebunden ist.

2. Nanokonjugat nach Anspruch 1, ferner umfassend ein Peptid mit endständiger Thioctsäure, das über mindestens eine Au-S-Bindung direkt an die Oberfläche des Goldnanopartikels gebunden ist, und wobei optional das Peptid mit endständiger Thioctsäure Bombesin mit endständiger Thioctsäure ist.

3. Nanokonjugat nach Anspruch 2, wobei das Thioctsäure-terminierte Bombesin eine Sequenz aufweist: Lipon-Gln-Trp-Ala-Val-Gly-His-Leu- Met-NH₂.

4. Nanokonjugat nach einem der Ansprüche 1 bis 3, wobei das Doxorubicin mit dem DTDTPA über eine Amidbindung verbunden ist.

5. Nanokonjugat nach einem der Ansprüche 2 bis 4, das durch ein Verfahren hergestellt wird, das das Konjugieren von AuNP-DTDTPA-Nanopartikeln mit dem Peptid mit endständiger Thioctsäure umfasst, wobei das Gold in dem Gold-Nanopartikel und das Peptid mit endständiger Thioctsäure in der Konjugationsreaktion in einem stöchiometrischen Verhältnis von etwa 1:0,5 bis etwa 1:4 vorhanden sind.

6. Nanokonjugat nach Anspruch 5, wobei das Gold in dem Goldnanopartikel und das Peptid mit endständiger Thioctsäure in der Konjugationsreaktion in einem stöchiometrischen Verhältnis von etwa 1:2 vorhanden sind.

7. Nanokonjugat nach einem der Ansprüche 1 bis 6, wobei das Peptid mit endständiger Thioctsäure etwa 1 bis etwa 40 Gew.-% des Nanokonjugats ausmacht.

8. Nanokonjugat nach Anspruch 7, wobei das Peptid mit endständiger Thioctsäure etwa 20 bis etwa 30 Gew.-% des Nanokonjugats ausmacht.

9. Nanokonjugat nach einem der Ansprüche 1 bis 8, wobei das Doxorubicin etwa 0,01 bis etwa 0,05 Gew.-% des Nanokonjugats ausmacht.

10. Nanokonjugat nach Anspruch 9, wobei das Doxorubicin etwa 0,03 Gew.-% des Nanokonjugats umfasst.

11. Nanokonjugat nach einem der Ansprüche 1 bis 10, wobei das Nanokonjugat einen hydrodynamischen Durchmesser von etwa 110 nm bis etwa 140 nm hat, gemessen durch dynamische Lichtstreuung (DLS).

12. Nanokonjugat nach Anspruch 11, wobei das Nanokonjugat einen hydrodynamischen Durchmesser von etwa 120 nm bis etwa 130 nm, gemessen durch dynamische Lichtstreuung (DLS), aufweist.

13. Nanokonjugat nach einem der Ansprüche 1 bis 12, wobei das Nanokonjugat einen Zetapotentialwert von etwa -15 mV bis etwa -30 mV aufweist.

14. Nanokonjugat nach Anspruch 13, wobei das Nanokonjugat einen Zetapotentialwert von etwa -20 mV bis etwa -25 mV aufweist.

15. Nanokonjugat nach Anspruch 1, umfassend: ein Peptid mit endständiger Thioctsäure, wobei das Peptid mit endständiger Thioctsäure über mindestens eine Au-S-Bindung direkt an die Goldnanopartikeloberfläche gebunden ist.

16. Pharmazeutische Zusammensetzung, umfassend das Nanokonjugat nach einem der Ansprüche 1 bis 15 und einen pharmazeutisch akzeptablen Träger.

17. Nanokonjugat nach einem der Ansprüche 1 bis 15 zur Verwendung bei der Behandlung einer Krebserkrankung, umfassend das Verabreichen einer therapeutisch wirksamen Menge des Nanokonjugats an eine Zielperson, die dessen bedarf.

18. Nanokonjugat nach Anspruch 17 zur Verwendung bei der Behandlung einer Krebserkrankung, wobei der Krebs aus der Gruppe ausgewählt ist, die aus akuter lymphatischer Leukämie (ALL), akuter myeloblastischer Leukämie (AML), Knochensarkom, Brustkrebs, Endometriumkrebs, Magenkrebs, Kopf- und Halskrebs, Hodgkin-Lymphom, Non-Hodgkin-Lymphom, Leberkrebs, Nierenkrebs, Multiples Myelom, Neuroblastom, Eierstockkrebs, Lungenkrebs, Weichteilsarkom, Thyome, Schilddrüsenkrebs, Blasenkrebs, Uterussarkom, Prostatakrebs, Dickdarmkrebs, Eierstockkrebs, nicht-kleinzelliger Lungenkrebs, Bauchspeicheldrüsenkrebs, Wilms-Tumor und Waldenström-Makroglobulinämie besteht.

19. Nanokonjugat nach Anspruch 17 oder Anspruch 18 zur Verwendung bei der Behandlung einer Krebserkrankung durch Injektion.

## Revendications

1. Nanoconjugué comprenant: une nanoparticule d'or (AuNP), de l'acide diéthylènetriamine pentaacétique dithiolé (DTDTPA), et de la doxorubicine, dans lequel le DTDTPA est directement lié à la surface de la nanoparticule d'or via au moins une liaison Au-S ; et la doxorubicine est liée au DTDTPA.

2. Le nanoconjugué de la revendication 1, comprenant en outre un peptide à terminaison acide thioctique directement lié à la surface de la nanoparticule d'or via au moins une liaison Au-S, et, optionnel, dans lequel le peptide à terminaison acide thioctique est la bombésine à terminaison acide thioctique.

3. Le nanoconjugué de la revendication 2, dans lequel la bombésine à terminaison acide thioctique a une séquence: Lipoïque-Gln-Trp-Ala-Val-Gly-His-Leu- Met-NH₂.

4. Le nanoconjugué de l'une quelconque des revendications 1 à 3, dans lequel la doxorubicine est liée au DTDTPA par une liaison amide.

5. Le nanoconjugué de l'une quelconque des revendications 2 à 4, qui est préparé par un procédé comprenant la conjugaison de la nanoparticule AuNP-DTDTPA avec le peptide à terminaison acide thioctique, dans lequel l'or dans la nanoparticule d'or et le peptide à terminaison acide thioctique sont présents dans la réaction de conjugaison dans un rapport stoechiométrique d'environ 1:0,5 à environ 1:4.

6. Le nanoconjugué de la revendication 5, dans lequel l'or dans la nanoparticule d'or et le peptide à terminaison acide thioctique sont présents dans la réaction de conjugaison dans un rapport stoechiométrique d'environ 1:2.

7. Le nanoconjugué de l'une quelconque des revendications 1 à 6, dans lequel le peptide à terminaison acide thioctique comprend environ 1% à environ 40% en poids du nanoconjugué.

8. Le nanoconjugué de la revendication 7, dans lequel le peptide à terminaison acide thioctique comprend environ 20% à environ 30% en poids du nanoconjugué.

9. Le nanoconjugué de l'une quelconque des revendications 1 à 8, dans lequel la doxorubicine comprend environ 0,01% à environ 0,05% en poids du nanoconjugué.

10. Le nanoconjugué de la revendication 9, dans lequel la doxorubicine comprend environ 0,03% en poids du nanoconjugué.

11. Le nanoconjugué de l'une quelconque des revendications 1 à 10, dans lequel le nanoconjugué a un diamètre hydrodynamique d'environ 110 nm à environ 140 nm tel que mesuré par diffusion dynamique de la lumière (DDL).

12. Le nanoconjugué de la revendication 11, dans lequel le nanoconjugué a un diamètre hydrodynamique d'environ 120 nm à environ 130 nm tel que mesuré par diffusion dynamique de la lumière (DDL).

13. Le nanoconjugué de l'une quelconque des revendications 1 à 12, dans lequel le nanoconjugué a une valeur de potentiel zêta d'environ -15 mV à environ -30 mV.

14. Le nanoconjugué de la revendication 13, dans lequel le nanoconjugué a une valeur de potentiel zêta d'environ -20 mV à environ -25 mV.

15. Le nanoconjugué selon la revendication 1, comprenant : un peptide à terminaison acide thioctique, dans lequel le peptide à terminaison acide thioctique est directement lié à la surface de la nanoparticule d'or via au moins une liaison Au-S.

16. Composition pharmaceutique comprenant le nanoconjugué de l'une quelconque des revendications 1 à 15, et un support pharmaceutiquement acceptable.

17. Le nanoconjugué de l'une quelconque des revendications 1 à 15 pour une utilisation dans le traitement d'un cancer comprenant l'administration d'une quantité thérapeutiquement efficace du nanoconjugué à un sujet qui en a besoin.

18. Nanoconjugué de la revendication 17 pour une utilisation dans le traitement d'un cancer, dans lequel le cancer est choisi dans le groupe constitué par la leucémie aiguë lymphoblastique (LAL), la leucémie aiguë myéloblastique (LAM), le sarcome osseux, le cancer du sein, le cancer de l'endomètre, le cancer gastrique, le cancer de la tête et du cou, le lymphome de Hodgkin, le lymphome non hodgkinien, cancer du foie, cancer du rein, myélome multiple, neuroblastome, cancer de l'ovaire, cancer du poumon, sarcome des tissus mous, thyomes, cancer de la thyroïde, cancer de la vessie, sarcome utérin, cancer de la prostate, cancer du côlon, cancer de l'ovaire, cancer du poumon non à petites cellules, cancer du pancréas, tumeur de Wilms et macroglobulinémie de Waldenström.

19. Le nanoconjugué de la revendication 17 ou de la revendication 18 pour une utilisation dans le traitement d'un cancer par injection.
